(19) Europäisches Patentamt
European Patent Office
Office européen des brevets

(11) Numéro de publication: 0 164 478
B1

# (12) FASCICULE DE BREVET EUROPÉEN

(45) Date de publication du fascicule du brevet: 25.01.89

(51) Int. Cl.⁴: **C 07 D 461/00**, A 61 K 31/475

(21) Numéro de dépôt: 84401983.6

(22) Date de dépôt: 04.10.84

## (54) Citrate de la (-) vinburnine et son procédé de fabrication.

(30) Priorité: 10.05.84 ES 532394

(43) Date de publication de la demande: 18.12.85 Bulletin 85/51

(45) Mention de la délivrance du brevet: 25.01.89 Bulletin 89/4

(84) Etats contractants désignés: CH DE FR LI

(56) Documents cité:
FR-A-2 085 630
FR-A-2 417 511

CHEMICAL ABSTRACTS, vol. 89, no. 23, 4 décembre 1978, page 653, no. 197770t, Columbus, Ohio, US; S.A. POGOSYAN et al.: "Indole derivatives. LX. 1,2,3,4,4a,5,7,8,13b,13c-Decahydro-13H-benz g indolo 2,3-a indolizines and 1,2,3,4,4a,5,7,8,9,14,14b,14c-dodecahydroisoindolol 1,2-a indolo 2,3-c azepines"

(73) Titulaire: COVEX (S.A.), Alberto Alcocer, 46, E-28016 Madrid (ES)

(72) Inventeur: Manresa, Maria Teresa, Martin Mora, 3, 28018 Madrid (ES)
Inventeur: Calvo, Fernando, Manzanal, S/N Soto Vinuelas, 28034 Madrid (ES)

(74) Mandataire: Viard, Jean, Cabinet VIARD 28 bis, avenue Mozart, F-75016 Paris (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 164 478 B1

LIBER, STOCKHOLM 1989

**Description**

La présente invention concerne un sel de (-) vinburnine présentant une solubilité dans l'eau améliorée par rapport à la vinburnine, et plus particulièrement destiné à des fins thérapeutiques, et son procédé de fabrication.

On a déjà proposé dans FR-A-2 085 630, de lutter contre les insuffisances de la circulation, et notamment de la circulation cérébrale, en utilisant les propriétés de la vincamone et de certains sels de cet alcaloïde.

FR-A-2 417 511 décrit également, dans le même but, des dérivés de la vincamone.

L'objet de la présente invention est de proposer un nouveau dédicament possédant des propriétés thérapeutiques améliorées.

Selon la présente invention, le sel de (-) vinburnine présentant une solubilité dans l'eau améliorée, et destiné à des fins thérapeutiques, est caractérisé en ce qu'il répond à la formule (I) suivante:

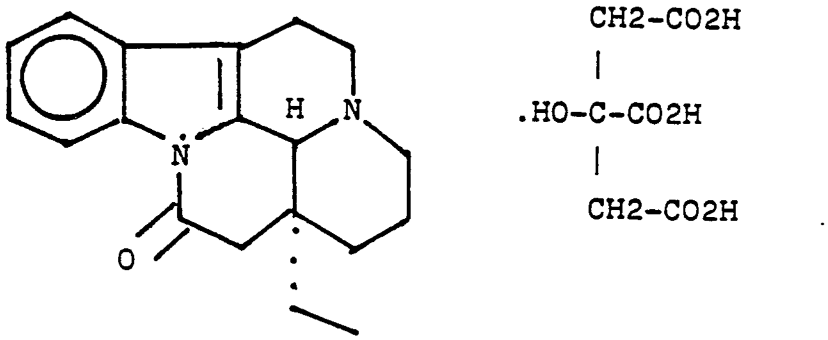

(I)

La vinburnine est un alcaloïde de la série de l'éburnane obtenu par semi-synthèse à partir de la vincamine ou de ses dérivés (FR-A-2 184 866) ou par synthèse à partir de la triptamine et autres précurseurs (BE-A-802 387; Bull. de la Société Chimie de France n° 11,12, 1975, p. 1962). La vinburnine présente une série de propriétés pharmacologiques intéressantes (Giorn. Geront. 1979, 27, 179; Acta Geront. 1977, 27; Eur. Neurol. 17 sup. 1, 175 1978) en relation avec la circulation cérébrale, puisqu'elle réactive le fonctionnnement de l'hématie, facilitant le transport et la libération de l'oxygène au niveau des tissus cérébraux. Elle réactive également le fonctionnement des neurones en reconstituant leur pôle énergétique et la synthèse de neurotransaminases. D'autre part, elle agit comme inhibiteur de l'agrégation des plaquettes et réduit la viscosité sanguine, minimisant la formation de micro-caillots dans le réseau capillaire.

Ainsi, la vinburnine améliore l'oxygénation cérébrale et la tolérance à l'anoxie des cellules cérébrales en augmentant la concentration de l'ATP dans les tissus cérébraux, lequel favorise la circulation cérébrale. D'autre part, le métabolisme aérobie du glucose augmente avec la diminution correspondante d'acide lactique.

C'est ce dernier point fut celui qui indiqua à la demanderesse à l'idée de préparer des sels de vinburnine qui améliorent sa propre action et qui facilite son absorption on a réalisé une série de sels, a partir des acides qui interviennent dans le métabolisme aérobie du glucose, desquels on a isolé le citrate de vinburnine (I) pour ses propriétés pharmacologiques, pour son absorption rapide par une partie de l'organisme et pour sa meilleure solubilité en solution aqueuse, ce qui permet de réaliser des préparations utilisables par voie orale en gouttes, ou injectables.

Les propriétés pharmacologiques du citrate de vinburnine ont été utlisées en thérapie avec de bons résultats. Les valeurs de DL50 ont été déterminées chez le chien par administration intraveineuse, et on a obtenu des valeurs sensiblement inférieures à celles correspondant à la vinburnine sous forme de base (voir tableau page 4).

L'invention sera mieux comprise à la lecture du complément de description qui suit se référant aux dessins annexés dans lesquels:

- La Fig. 1 est une illustration graphique de l'augmentation de consommation cérébrale de desoxyglucose chez un animal traité avec le citrate de vinburnine;
- La Fig. 2, est une illustration graphique de l'augmentation de la dissociation de l'oxyhémoglobine chez un animal traité avec le citrate de vinburnine.

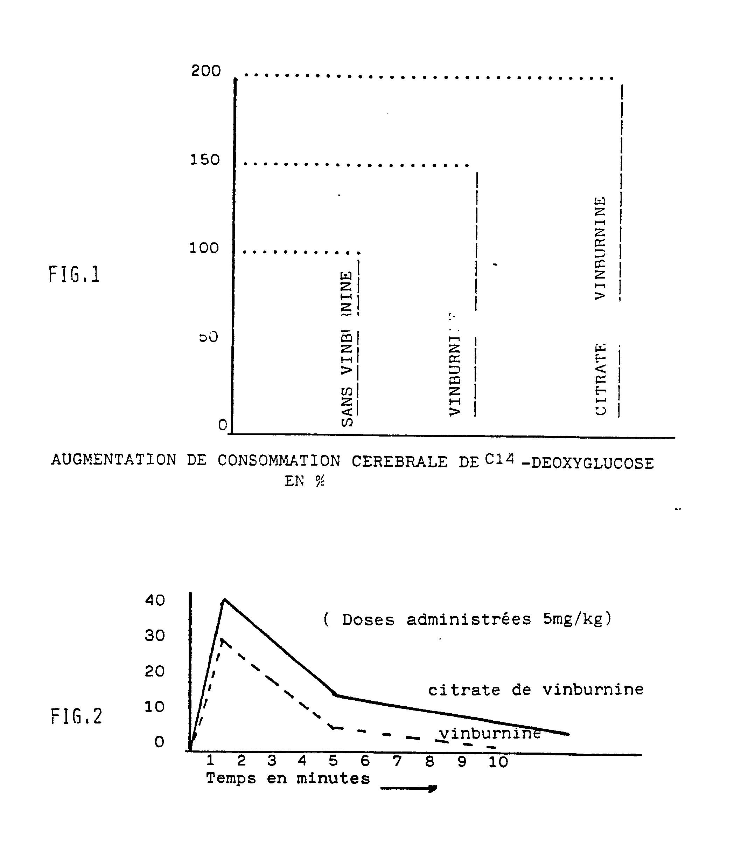

Dans l'étude sur l'augmentation de la consommation du glucose, on a utilisé des doses de 1 à 4 Mg/kg de citrate de vinburnine chez des chiens auxquels on administre du deoxyglucose marqué au C14, par voie orale. On peut voir sur le graphique de la Fig. 1 l'importance de l'augmentation de la consommation cérébrale du C14-desoxyglucose chez un animal traité avec la vinburnine et avec le citrate de vinburnine, par rapport à un animal non traite.

On constate, d'après ce graphique, qu'il y a une consommation du glucose plus importante lorsque l'animal est traité avec du citrate de vinburnine. Ainsi, on observe une augmentation dans la dissociation de l'oxyhémoglobine quand on administre 5 mg/kg de citrate de vinburnine à des chiens au préalable anesthésiés. Cette différence observée dans l'importance de la dissociation de l'oxyhémoglobine par la vinburnine et le

citrate de vinburnine est illustrée sur le graphique de la Fig. 2.

Les graphiques représentés sur les Figs 1 et 2 laissent apparaître que le citrate de vinburnine est un oxygénateur cérébral plus efficace que la vinburnine.

D'autre part, on a réalisé une étude sur la pression sanguine et la circulation cérébrale en administrant des doses intraveineuses de 1 à 4 mg/kg d'animal.

Dans l'étude de l'effet de ce compose, on a enregistré et calculé la circulation cérébrale (artère carotide interne) et la résistance vasculaire lors de l'administration des composés par voie intraveineuse et intraartérielle chez des chiens. Les valeurs données dans le tableau suivant sont rapportés à la valeur de 1,0 donnée arbitrairement à la vinburnine.

**TABLEAU**

| COMPOSE | DL50 | CERVEAU | | | |
|---|---|---|---|---|---|
| | | augmentation circulation | | Diminution résistance vasculaire | |
| | | i.v. | i.a. | i.v. | i.a. |
| VINBURNINE | 1,0 | 1,0 | 1,0 | 1,0 | 1,0 |
| CITRATE DE VINBURNINE | 1,2 | 0,9 | 0,8 | 0,8 | 0,6 |

Les résultats reflétés dans le tableau indiquent que le citrate de vinburnine produit une augmentation effective de la circulation cérébrale.

En outre, alors que la vinburnine est un produit insoluble dans l'eau ce qui permet difficilement de préparer des solutions aqueuses de celle-ci, utilisables par voie orale en gouttes ou injectables, le citrate de vinburnine est soluble en solution aqueuse, ce qui permet son utilisation par voie orale.

Le procédé de préparation du citrate de vinburnine selon l'invention sel est caractérisé en ce que l'on fait agir la (-) vinburnine pure avec une solution hydroalcoolique ou alcoolique d'acide citrique.

Selon le procédé, la solution réactionnelle est soumise à une température de 20° C pendant une période de 5 à 10 minutes avec agitation énergique. Une fois ce temps écoulé, lorsque la réaction est effectuée en milieu alcoolique, la solution se concentre à la moitié de son volume initial, et l'on obtient, après refroidissement à une température de 0° C un précipité de citrate de vinburnine qui est filtré, lavé et laissé sécher à l'air.

Le citrate de vinburnine peut également être préparé à partir d'autres sels de (-) vinburnine comme le phosphate (II)

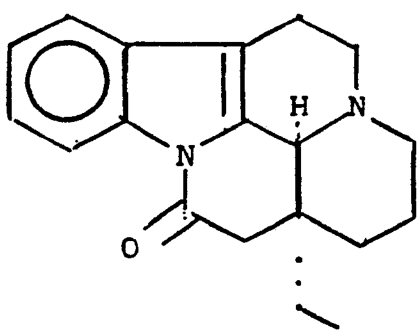

. PO4H3

(II)

en le faisant réagir avec le sel sodique ou potassique de l'acide citrique.

L'avantage de ce procédé consiste à pouvoir préparer d'une manière simple, peu coûteuse, avec de bons rendements, le citrate de (-) vinburnine (I) à des fins thérapeutiques. D'autres caractéristiques et avantages de l'invention apparaîtront à la lecture des exemples de préparation du citrate de (-) vinburnine par le procédé conforme à l'invention, donnés ci-après, à titre illustratif et non limitatif.

**EXEMPLE I**

0,65 g d'acide citrique est dissous dans 50 ml d'éthanol absolu et on ajoute 1 gr de (-) vinburnine. Le mélange est agité pendant 10 minutes au bout desquelles la solution se concentre à la moitié du volume initial et est refroidi à 0°C ce qui provoque la précipitation du citrate. On filtre le précipité, on le lave avec 2 ml d'alcool absolu et on le sèche à l'air en absence de lumière. Le produit est le citrate de (-) vinburnine (1) avec point de fusion entre 202 et 204° C (rendement: 90 %).

**EXEMPLE II**

20 g de la (-) vinburnine sont mélangés à 50 ml d'éthanol (99 %) en ajoutant 8,6 g d'acide phosphorique à 85 %. Le mélange est agité pendant quelques minutes au bout desquelles on filtre la solution qu'on laisse ensuite refroidir. Le phosphate précipite et on filtre le précipité qu'on lave à l'éthanol. Le produit obtenu est la phosphate de (-) vinburnine (II) avec point de fusion à 243,5° C (rendement 90 %).

**EXEMPLE III**

0,6 grs de citrate sodique est mélangé à 50 ml d'alcool absolu et on ajoute immédiatement 1 gr de phosphate de (-) vinburnine (II). Le mélange est ajouté pendant 15 minutes et le volume se réduit de moitié on refroidit à 0° C ce qui provoque la précipitation du citrate dont la structure correspond au composé de l'exemple 1 (rendement 92 % - point de fusion 203 - 204° C).

**EXEMPLE IV**

8,6 g de citrate de la (-) vinburnine obtenu selon les exemples 1 et 2, sont dissous dans un litre d'eau distilée en ajustant le pH de la solution entre 5 et 5,5; 2,5 ml de cette solution contiennent 30 mg de la (-) vinburnine base qui correspond à la dose thérapeutique recommandée.

**EXEMPLE V**

3,6 grs d'acide citrique sont dissous dans 50 ml d'éthanol absolu et 50 ml d'eau distillée. A cette solution, on ajoute 5 grs de la (-) vinburnine. On homogénéise la solution à la température de 20° C et on ajuste son pH dans l'intervalle 5 - 5,5. La solution hydroalcoolique est portée à un volume de 1000 ml. avec de l'eau distillée en maintenant son pH à 5 - 5,1. On laisse la solution reposer 24 heures au bout desquelles on la filtre et 2,5 ml de cette solution contiennent 30 mg de la (-) vinburnine base ce qui correspond à la dose thérapeutique recommandée.

**Revendications**

1. Sel de (-) vinburnine présentant une solubilité dans l'eau améliorée et plus particulièrement destiné à des fins thérapeutiques caractérisé en ce qu'il répond à la formule (I) suivante

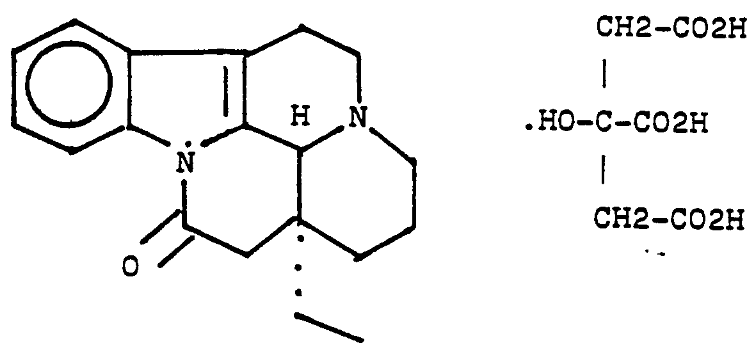

(I)

2. Procédé de préparation du citrate de (-) vinburnine de formule (I) caractérisé en ce que l'on fait réagir en milieu alcoolique ou hydroalcoolique, la (-) vinburnine avec l'acide citrique à une température de l'ordre de 20° C durant 5 à 10 minutes pour donner le citrate de (-) vinburnine et en ce que l'on porte avec de l'eau la solution réactionnelle à un volume déterminé de telle façon que la solution résultante contienne 30 mg de (-) vinburnine base pour 2,5 ml de solution, le pH de ladite solution étant compris entre 5 et 5,5.

3. Procédé pour la préparation de citrate de (-) vinburnine selon la revendication 2, caractérisé en ce que l'on

fait réagir en milieu alcoolique ou hydroalcoolique un sel de la (-) vinburnine, tel que le phosphate de vinburnine avec le citrate sodique ou potassique, à une température de 20°C durant 5 à 10 minutes pour donner le citrate de (-) vinburnine et en ce que l'on porte avec de l'eau la solution réactionnelle à un volume déterminé de telle façon que la solution aqueuse résultante contiennent 30 mg de (-) vinburnine base pour 2,5 ml de solution, le pH de ladite solution étant compris entre 5 et 5,5.

4. Procédé de préparation du citrate de la (-) vinburnine selon l'une quelconque des revendications 2 et 3, caractérisé en ce que la réaction est effectuée dans l'éthanol absolu et en ce que l'on obtient le citrate de (-) vinburnine par concentration de la solution réactionnelle à la moitié de son volume puis, par refroidissement de ladite solution à une température de l'ordre de 0°C, ce qui provoque la formation d'un précipité correspondant au produit recherché.

5. Procédé de préparation du citrate de la (-) vinburnine selon l'une quelconque des revendications 2 et 3, caractérisé en ce que la réaction se produit dans un mélange d'éthanol et d'eau à 50 % à la température de 20°C.

**Patentansprüche**

1. Salz von (-) Vinburnine mit verbesserter Wasserlöslichkeit, das insbesondere für therapeutische Zwecke bestimmt ist,
dadurch gekennzeichnet,
daß es gemäß nachfolgender Formel (I) reagiert.

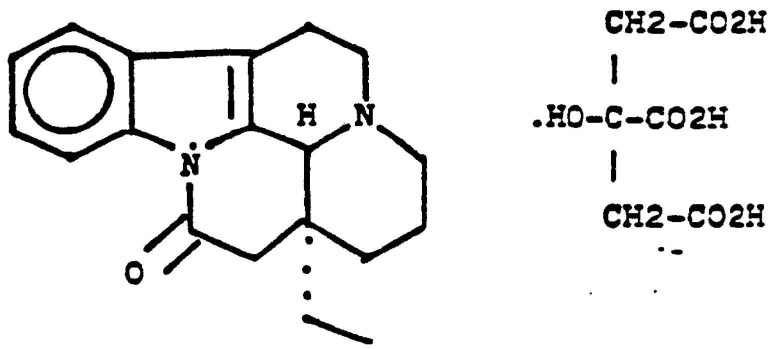

2. Verfahren zum Herstellen des (-) Vinburnine-Zitrats nach Formel (I),
dadurch gekennzeichnet, daß man es in alkoholischem oder hydroalkoholischem Mittel reagieren läßt, und zwar das (-) Vinburnine während fünf bis zehn Minuten mit Zitronensäure bei einer Temperatur in der Größenordnung von 20°C, um das (-) Vinburnine-Zitrat auszufällen, sowie gekennzeichnet dadurch, daß man die Reaktionslösung mit Wasser so auf ein bestimmtes Volumen bringt, daß die Endlösung 30 mg (-) Vinburninesole auf 2,5 ml Lösung enthält und der ph-Wert der besagten Lösung zwischen 5 und 5,5 liegt.

3. Verfahren zum Herstellen des (-) Vinburnine-Zitrats gemäß Anspruch 2, dadurch gekennzeichnet, daß man ein (-) Vinburnine-Salz in alkoholischem oder hydroalkoholischem Mittel reagieren läßt, und zwar das Vinburnine-Phosphat mit soda- oder kalihaltigem Zitrat während fünf bis zehn Minuten bei einer Temperatur von 20°C, um das (-) Vinburnine-Zitrat auszufällen, und dadurch gekennzeichnet, daß man mit Wasser die Reaktionslösung so auf ein bestimmtes Volumen bringt, daß die wässrige Endläsung 30 mg (-) Vinburnine auf 2,5 ml Lösung enthält und der ph-Wert der besagten Lösung zwischen 5 und 5,5 liegt.

4. Verfahren zum Herstellen von (-) Vinburnine gemäß einem der Ansprüche 2 und 3, dadurch gekennzeichnet, daß die Reaktion in reinem Methanol durchgeführt wird und daß man das (-) Vinburnine-Zitrat erhält durch Konzentration der Reaktionslösung auf die Hälfte ihres ursprünglichen Volumens infolge Abkühlung der Lösung auf ein Temperaturniveau von etwa 0°C, was zur Bildung eines Niederschlages führt, der dem gesuchten Produkt entspricht.

5. Verfahren zum Herstellen des (-) Vinburnine-Zitrats gemäß einem der Ansprüche 2 und 3, dadurch gekennzeichnet, daß die Reaktion in einer Mischung aus 50°C Äthanol und Wasser bei einer Temperatur von 20°C abläuft.

**Claims**

1. A salt of (-) vinburnine having improved solubility in water, and intended more particularly for tnerapeutic purposes, characterized in that it has the following formula (I):

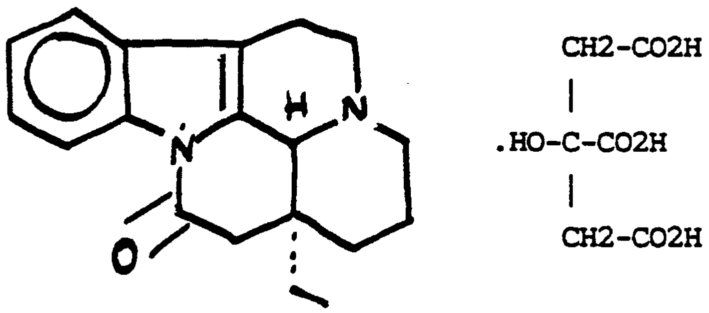

(I)

2. A method of preparing (-) vinburnine citrate according to formula (I), characterized in that the (-) vinburnine is caused to react with citric acid in an alcohol or a water-alcohol medium at a temperature of about 20°C for 5 to 10 minutes in order to obtain the (-) vinburnine citrate, and in that the reaction solution is made up with water to a volume which is determined in such a manner that the resulting solution contains 30 mg of (-) vinburnine base per 2.5 ml of solution, with the pH of said solution lying in the range 5 to 5.5.

3. A method of preparing (-) vinburnine citrate according to claim 2, characterized in that a salt of (-) vinburnine, such as vinburnine phosphate, is caused to react with sodium or potassium citrate in an alcohol or a water-alcohol medium at a temperature of 20°C for 5 to 10 minutes in order to obtain (-) vinburnine citrate, and in that the reaction solution is made up with water to a volume determined in such a manner that the resulting aqueous solution contains 30 mg of (-) vinburnine base per 2.5 ml of solution, with the pH of said solution lying in the range 5 to 5.5.

4. A method of preparing (-) vinburnine citrate according to claim 2 or 3, characterized in that the reaction is performed in absolute ethanol and in that (-) vinburnine citrate is obtained by concentrating the reaction solution to one half of its volume, then by cooling said solution to a temperature of about 0°C, thereby causing a precipitate of the desired product to be formed.

5. A method of preparing the (-) vinburnine citrate according to claim 2 or 3, characterized in that the reaction tis place in a 50 % mixture of ethanol and water at a temperature of 20°C.

FIG.1

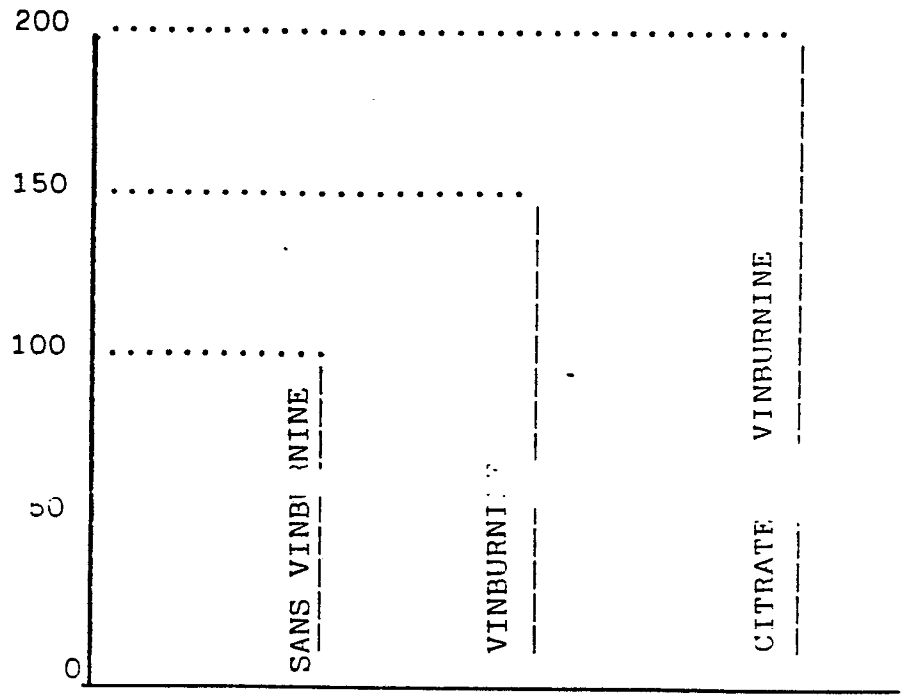


AUGMENTATION DE CONSOMMATION CEREBRALE DE C14 -DEOXYGLUCOSE
EN %

FIG.2